# EUROPEAN PATENT SPECIFICATION

(11) **EP 0 402 023 B1**
(45) Date of publication and mention of the grant of the patent: **26.10.1994**
(21) Application number: 90305747.9
(22) Date of filing: 25.05.1990
(51) Int. Cl.: G01N 33/543, G01N 33/569

(54) **Elongated membrane flow-through diagnostic device and method**
Diagnostisches Gerät mit verlängerter Durchflussmembran und Verfahren
Dispositif diagnostique de courant de membrane allongée et méthode

(30) Priority: 26.05.1989 US 358786
(43) Date of publication of application: 12.12.1990
(73) Proprietor: E-Y LABORATORIES, INC., San Mateo California 94401 (US)
(72) Inventor: Chu, Albert E., Hillsborough, CA 94010 (US); Chun, Peter K., South San Francisco, CA 94080 (US); Yeung, Siu Chin C., San Mateo, CA 94401 (US)
(74) Representative: Allard, Susan Joyce

(56) References cited:
- EP-A- 0 310 406
- ANALYTICAL BIOCHEMISTRY, vol. 137, 1984, Academic Press Inc.; J.M. KITTLER et al., pp. 210-216#
- JOURNAL OF MICROBIOLOGY, vol. 26, no. 1, 1988; BLOMBERG et al., pp. 111-115#

## Description

The present invention relates to devices and methods for analyte assay in liquid samples. More particularly, the invention relates to devices and methods utilizing immobilized specific binding receptors for analytes in liquid samples derived from biological specimens.

There has long been an interest in the development of assay systems which can determine the presence or amount of specific substances in samples derived from biological specimens. Over the past two decades, immunoassays, which employ naturally occurring receptors directed to specific target substances, have provided valuable diagnostic tools for detecting substances of clinical significance. There are numerous immunoassays in the prior art in which one component of an immunological pair, e.g., an antigen or antibody, is detected or measured by using the complementary partner labelled with a tag which provides a detectible signal.

In one assay technique, known as a competitive binding technique, the substance to be detected competes with a labelled reagent of the same substance for a limited number of receptor sites. For example, for the detection of an unknown amount of a selected antigen in a liquid sample, a known amount of the labelled antigen is added to the sample and then contacted with receptor antibody specific for the antigen. The amount of labelled antigen which binds to the antibody is inversely proportional to the amount of the unknown antigen in the sample.

In another assay, known as a sandwich assay, receptor antibody is bound to a solid surface and the selected antigen in the sample binds to that antibody. A second labelled antibody capable of binding to the bound antigen is then reacted with the antigen to form an immobilized reaction product. The label in the reaction product is detected as an indication of the presence of the antigen in the sample.

For the detection or measurement of an antigen using a sandwich technique, antisera have been used for many years for both the labelled antibody and for the receptor antibody on the surface. More recently, monoclonal antibodies have been used in place of the antisera in such assay. In one such system, described in Wada, et al., Clin. Chem., 28(9):1986-1966 (1982), the receptor antibody was directed to one subunit of a particular antigen, hCG, while an enzyme-labelled monoclonal antibody was directed to another subunit. In this assay, the receptor antibody is immobilized on the inside of the test tube to which the sample was added.

Reaction on a solid surface can be relatively slow because the contact between the immobilized reagent and the analyte in the sample is limited. The assay time has been reduced by immobilizing the receptor antibody within a porous membrane, exposing the antibody molecules in a three-dimensional matrix. In many such systems the liquid sample containing the target antigen is drawn through the membrane into an underlying absorbent material. One such system, disclosed for use in a competitive binding assay, is U.S. Patent No. 3,888,629. Other systems disclosed for use in competitive or sandwich assays include U.S. Patent Nos. 4,246,339 and 4,366,241.

Also EP-A-0 310 406 discloses a device comprising a porous reaction membrane, adsorbent means and a well bounded by a liquid retaining wall shaped to receive the sample and facilitate contact with the membrane.

It is known to immobilize an antibody onto a membrane to bind an antigen and detect it, e.g. in a sandwich assay. The sample solutions and reagents flow through the membrane into an absorbent material on the other side of the membrane. One such system is described in U. S. Patent No. 4,632,901. However, there is no suggestion that this type of system could be utilized for the detection of protein blots.

Protein blotting is a term used to describe the transfer of electrophoretically-resolved biological protein samples to an immobilizing matrix followed by a detection. Where the biological specimen is a protein, the blot is referred to as a Western blot. The general techniques for separations of the proteins and for blotting on the immobilized phase are well known (Techniques in Molecular Microbiology, J. Walker and W. Gaastre (Eds); G. Bers and D. Garfin, Bio Techniques, Vol. 3, No. 4, pp.276-288 (1985); Journal of Immunological Methods, 100 (1987) 281-282). As described, polyacrylamide gel electrophoresis is one suitable technique for this separation. Blotting matrices are available including nitrocellulose, DBM and DPT paper and ion exchange papers (e.g. DEAE). Nitrocellulose is a particularly effective blotting membrane. In a typical application, the proteins migrate through the gel under the influence of an electric field. The rate of migration is dependent on the charge, size and shape of the protein. The protein in the gel is then electrophoretically transferred to a support medium such as the nitrocellulose membrane.

After the blotting process, the protein on the membrane is detected in any of the aforementioned techniques. Commonly, antibody and various labelled materials such as an enzyme conjugate or a complex of colloidal gold with antibody has been used to detect the protein. It is known that the specificity of blotting may be difficult to control to avoid nonspecific binding to the membrane. Accordingly, a variety of blocking agents have been applied to the membrane prior to addition of the sample and labelled antibody. A common effective blocking agent is the surfactant Tween-20. Such blocking agent is usually added at a low concentration, e.g. 0.05% Tween-20.

Strips of nitrocellulose containing electrophoretically separated HIV-1 viral protein are commercially available for the detection of antibodies in an AIDS patient's serum. In such techniques, the strips are immersed into a solution of serum sample and labelled second antibody and mechanically agitated during incubation. Wash steps are normally required between incubations. Assay procedures of this type, termed overlays, typically require several hours. Thus, the procedure is labor and apparatus intensive.

Moreover, incubating such protein containing nitrocellulose strips with continuous agitation cause elution from the membrane of substantial portions of the blotted proteins. An other problem with this overlay approach is that the protein blot is surrounded by an unstirred layer (Nernst) of solvent which restricts diffusion in mass transport across the liquid solid interface. This causes the antibody to be is bound more quickly than it can be replenished by diffusion from the bulk solution. Thus, the observed rate constant is slower than expected and a slower reaction occurs.

A variety of label reagents have been used for anti-HIV immuno-blotting assays. Typically, such assays use a secondary antibody for visualizing antigen-bound primary antibodies. While enzymes have been used, gold labelled secondary antibodies have also been employed.

This invention provides an assay device and method for detecting an analyte in a liquid sample, particularly a sample derived from biological specimen.

In accordance with one aspect of the invention there is provided a storage and reaction apparatus for use in assays for the detection and/or determination of a bindable target substance in a liquid sample suspected of containing such substance, characterised in that it comprises
(a) a liquid-permeable, porous reaction membrane having an upper and lower surface, at least said upper surface having immobilized thereon a receptor capable of directly or indirectly binding to the bindable substance, said receptor comprising a protein blot along said strip
(b) a body of absorbent material capable of absorbing liquid, said body having a surface located adjacent to the lower surface of the reaction membrane, and
(c) container means for said reaction membrane and absorbent material, including a top wall defining a fluid port adjacent said receptor and including fluid seal means disposed at the periphery of the fluid port defining a seal between said container top wall and reaction membrane, said top wall and membrane defining an open space to the periphery of said seal means.

In accordance with another aspect of the invention there is provided an assay for detection and/or determination of bindable target substance in a liquid sample using the storage and reaction apparatus as described above, the assay comprising applying the sample, a visable labelled substance and any additional reagents to the upper surface of said permeable reaction membrane whereby the applied liquid will permeate through said fluid port and the membrane lower surface into said absorbent body, and permeable reaction membrane whereby the applied liquid will permeate through said fluid port and the membrane lower surface into said absorbent body, and thereafter detecting said labelled substance bound to the upper surface as an indication of the presence or absence of bindable target substance bound to said receptor of said upper surface.

The liquid sample is assayed to form a detectible reaction product on the membrane. In preferred assay embodiments, the liquid sample is biologically derived (e.g., urine or serum) and is suspected to include as the target substance, an antibody capable or being bound by the receptor immobilized on the membrane.

In the present invention, the receptor on the membrane is a protein blot, and the bindable target substance in the liquid sample comprises antibody to said receptor. More specifically, a preferred antigen is an HIV viral protein.

The apparatus includes an elongate fluid port with a fluid seal disposed at the periphery of the fluid port defining a seal between a container wall and reaction membrane. Preferably, the fluid seal means is a continuous rim projecting from the container wall towards the membrane strip. A preferred membrane is nitrocellulose, more particularly, paper-backed nitrocellulose.

A preferred feature of the invention is the application of a blocking solution to the reaction membrane prior to the addition of the sample. In particular, a preferred blocking solution including between about 1% and about 2% Tween-20® surfactant in combination with bovine serum albumin is particularly effective.

The invention will now be described by way of example with reference to the accompanying drawings in which:
Figure 1 is an exploded view of an assay device of the present invention,
Figure 2 is a top view of the assay device of Figure 1,
Figure 3 is an expanded cross-sectional view of the assay device of Figure 2 taken along the line 3-3,
Figure 4 is an enlarged view of a portion of the assay device of Figure 3 illustrating the membrane and seal in detail, and
Figure 5 is another embodiment of an assay device according to the invention.

The present invention provides an improved assay device and method for detecting an analyte or target substance in a fluid sample, particularly a sample derived from a biological specimen. More specifically, the system is useful for the detection of antibody in the serum of a patient, e.g. one suspected of having AIDS, using a protein blot of viral or recombinant HIV protein, or a portion of the same, such as ENV 9 envelope protein (GP 120 and parts of GP 41 of the HIV virus).

The device of the present invention serves the combined function of storing liquid reagents and permitting their reaction on a membrane and detecting the target substance. It includes a porous reaction membrane containing or impregnated with an immobilized receptor, which is a protein blot, which is capable of directly or indirectly binding to the target substance. The device also includes a body of absorbent material located below and supporting to the porous membrane. The device is formed into a box-like container including a top wall defining a fluid port adjacent to the receptor and including a fluid seal, preferably a rim, disposed at the periphery of the fluid port.

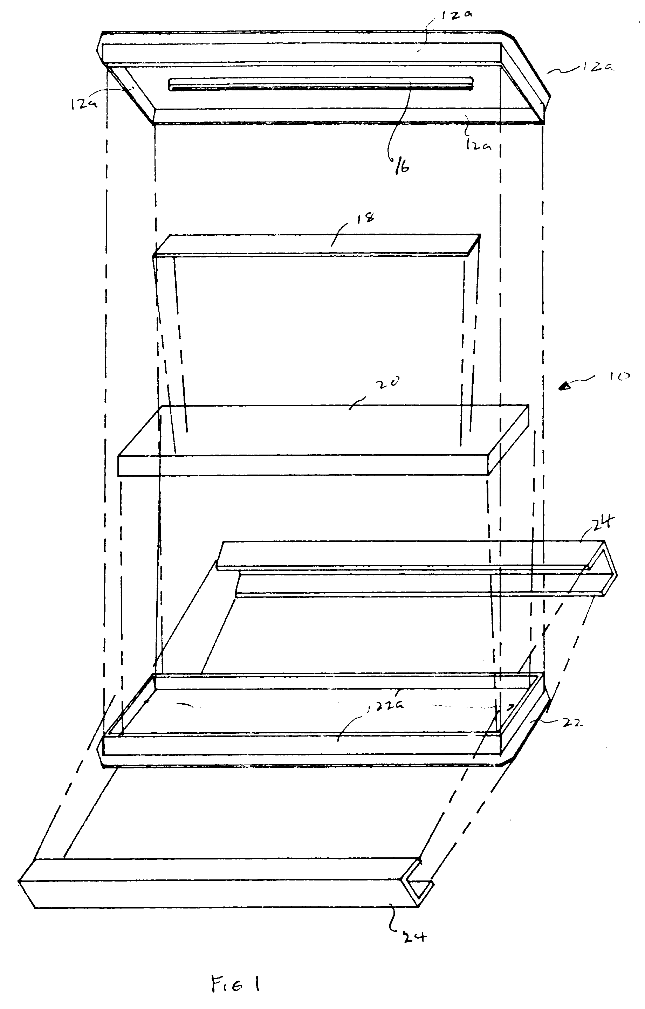

Referring to the drawings, Figure 1 depicts a representative assay device 10 according to the present invention which may be disposable or reusable with appropriate cleaning. While the device is illustrated in a rectangular shape, other appropriate shapes may be employed so long as it includes an appropriate port for receiving and temporarily restoring the liquid.

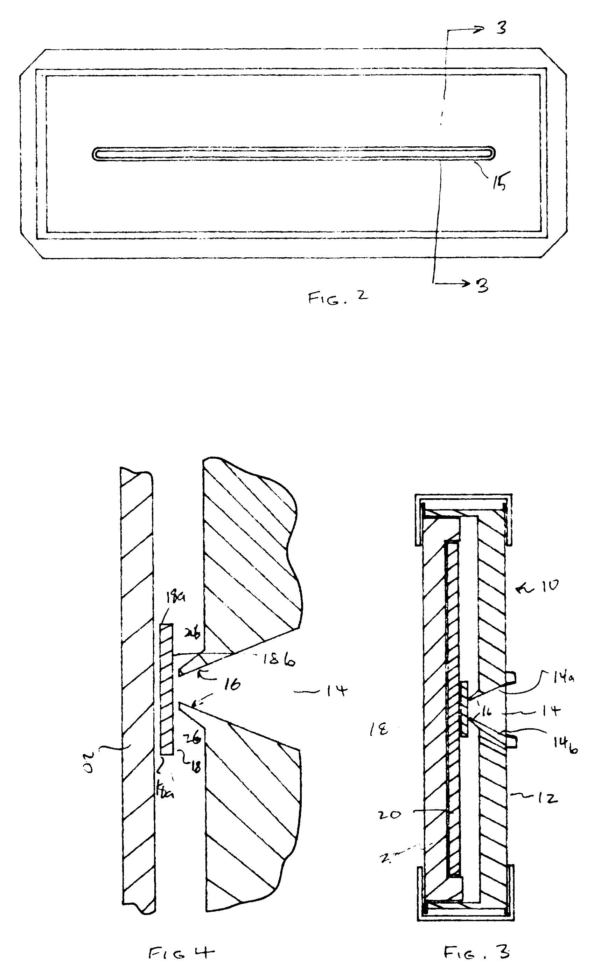

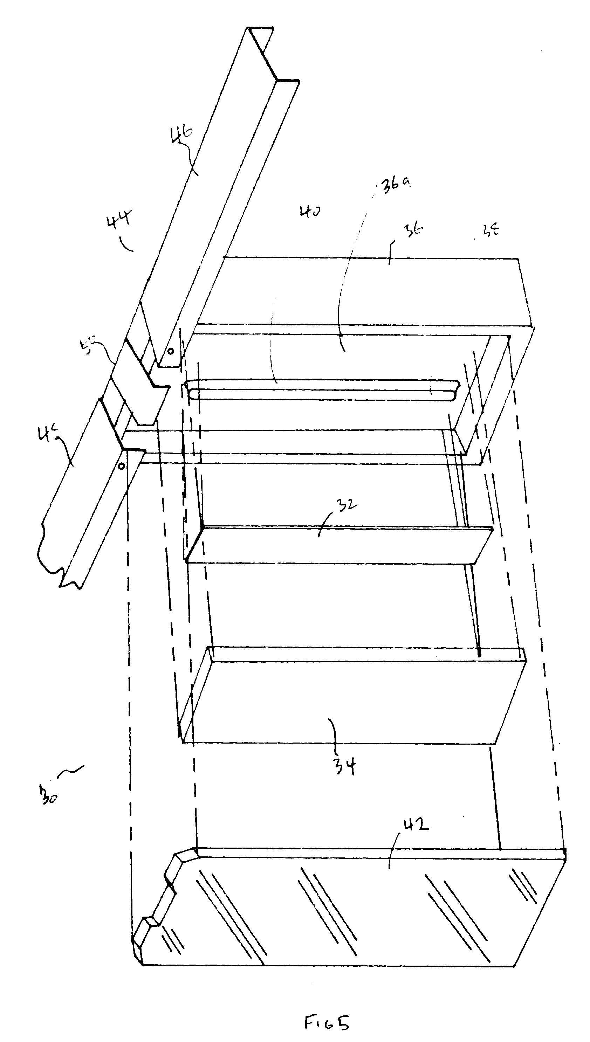

Referring to Figures 1-4, the device of the present invention is illustrated in a form for ready assembly and disassembly. The device generally, designated by the number 10, includes a top wall 12 with a fluid port 14, best illustrated in Figures 3 and 4, is defined by parallel elongate side walls 14a and 14b which slope inwardly from the top to the bottom of the top wall. An upwardly projecting rim 15 is provided at the periphery of the port to contain any liquid applied to port 14 in excess of a volume which would be contained in that portion of port 14 at the level of the top wall.

Sealing means in the form of a continuous rim 16 of downwardly projecting walls 16 provided on the bottom side of top wall 12. The sealing means is disposed at the periphery of port 14 and defines a seal between the bottom of container top wall 12 and reaction membrane 18 as described below. As illustrated, such sealing means is in the form of an integral continuous rim 16 extending around the outlet of port 14. If desired, other sealing means may be employed so long as liquid flowing through port 14 continues to flow through the membrane and into absorbent material body 20 without leaking around the sealing means.

As illustrated in Figures 1 and 3, the device also includes a bottom wall 22 with perpendicular upwardly projecting side wall 22a defining an enclosure for absorbent material 20. Side walls 12a of top wall 12 fit over upwardly projecting side walls 22a in close tolerance. Top wall 12 and bottom wall 22 form in combination a box-like enclosure sealed against liquid except through port 14.

Clamping means is provided as a safety measure to retain top wall 12 and bottom wall 22 in contact with each other in a fluid enclosing relationship. As illustrated, such clamping means comprises elongate U-shaped members 24 which slide over walls 12 and 22 retaining the same in a box-like enclosure. Components 12, 22, 24 are suitably formed of a disposable plastic material such as polycarbonate, polyethylene, polypropylene or polyvinyl chloride or of a durable material such as a metal (e.g. aluminum) which would permit reuse of device 10 if desired.

As illustrated, membrane means 18 includes a porous reaction membrane with an externally visible upper surface. The reaction membrane may be of any type capable of immobilizing reaction product of the reagents and sample component without adversely affecting the reaction, and permits passage of the remainder of the liquid sample or of a washing solution. Suitable membranes may be formed of any material capable of immobilizing the blotted protein employed in the practice of the present invention, such as nylon, glass fibers, or other natural or synthetic materials which can be coupled directly or indirectly to the selected receptor. The porosity of the membrane preferably varies from about 0.2 to about 12 microns.

However, as a presently preferred embodiment, membrane means 18 comprises paper-backed nitrocellulose, or other types of nitrocellulose membranes with similar characteristics. This embodiment comprises a nitrocellulose membrane backed with porous paper similar to filter paper. A representative example is commercially available under the tradename BAC-T-KOTE by Schleicher and Schuell. This preferred membrane is substantially more durable than nitrocellulose alone and can be employed without any other support component. Also, it provides enhanced sensitivity to the reaction. Also, polyester supported nitrocellulose may be used such as supplied under the name NITROPLUS by Micron Separation, Inc.

As described below, a binding reagent which is a blotted protein is first immobilized on the membrane. The reagent reacts with and captures the predetermined target substance of the liquid sample to be assayed. An immunological protein such as an antigen, can be immobilized directly or indirectly onto such membranes, such as nitrocellulose, by either adsorption or by covalent bonding.

The depth or thickness of the membrane is selected so that an adequate amount of binding reagent i.e. blotted protein can be immobilized to capture the sample component. However, the thickness should not be so great as to cause undue delay of the passage of the liquid sample through the membrane.

The absorbent body 20 of the present device can employ any of the known and conventionally employed absorbent materials which serve to draw liquid through a porous membrane, such as, for example, by capillary action. Useful known materials include cellulose acetate fibers, polyester, polyolefin or other such materials. It has also been found convenient to use layers of commercial available filter paper, or even toilet paper, which can be selected to provide sufficient volume to absorb the liquid employed during the assay of the present invention.

In the device of the present invention, a flow-through system is provided suitable for a Western blot analysis of a patient's serum for antibody. This has a number of significant advantages over conventional Western blot overlay techniques, not the least of which is the ability to perform the test in less than an hour, more specifically, in five to ten minutes or less. Furthermore, the test is easy to perform by lab technicians.

As illustrated, rim 16 projects from container top 10 towards reaction membrane 18 which is supported by absorbent body 20. When the unit is closed as illustrated in Figure 3 and ready for the application of sample, rim 16 is pressed against the top surface of membrane 18 under sufficient compression to prevent any substantial leakage between the walls of rim 16 and the reaction membrane. An open space 26 is defined between the bottom of top wall 12 and the top of absorbent body 20 to the periphery of rim wall 16. This has been found to prevent leakage around rim 16, causing the liquid to flow through port 14 and the exposed area of membrane 18 rather than flowing along the top surface of membrane 18 and leaking through the contact points with rim 16. Preferably, rim 16 contacts membrane 18 along the length of the strip to the interior of the membrane side walls 18a. As illustrated, rim walls 16 provide a rim with a maximum thickness of less than about 5mm and preferably less than about 3mm at the contact point with membrane 18. Also, as illustrated, such contact point leaves a substantial portion of the top surface 18b of membrane 18 to the exterior of the contact point providing open space 26. As illustrated, such open space comprises a greater area than that portion of the upper membrane exposed to the port 14.

An alternative form of the assay device of the present invention is illustrated in Figure 5. That device is functionally equivalent to the device of Figure 1-4 but is more substantial in construction lending itself to many repeated uses. As the device, generally designated by the number 30, includes membrane 32 similar to membrane 18 and absorbent body 34 similar to absorbent body 20 of Figures 1-4. The top wall 36 is of rectangular shape and substantial thickness, suitably formed of a rigid plastic material. Top wall 36 defines a port 38 with a downwardly projecting rim 40 serving as sealing means similar to downwardly projecting rim 16 of Figures 1-4. Top wall 38 is undercut to define recess 36a in which absorbent material 34 is nested.

The device also includes a flat bottom wall 42 also suitably formed of rigid plastic. Absorbent material 34 rests on bottom wall 42.

Clamping means 44 is provided to form the unit into a generally fluid-tight rectangular box. Clamping means 44 include U-shaped arms 46 and 48 which are mounted for pivotal movement to top wall 36. Walls 46 and 48 retain the unit in a box-like configuration when closely adjacent to top wall 36 and bottom wall 42. An optional additional clamp 50 is also mounted to top wall 36 extending downwardly therefrom including an angle piece into which bottom wall 42 slides. In the illustrated embodiment, clamping means 44 is formed of metal while top and bottom walls 36 and 42 are formed of a durable plastic material.

The system is applicable to an immunoassay wherein the sample component is one component of an immunological pair as defined below. The immunological pair includes two components which immunologically bind to each other. Specific immunological pairs include antigens and their antibodies (monoclonal antibodies or polyclonal antibodies), biologically functional haptens and their antibodies, enzymes and their substrates, hormones and their receptors, vitamins and their receptors, biotin and either avidin or an antibody to biotin, and lectin and its specific binding mono, di-or trisaccharide or glycoprotein.

For simplicity of description, the system will be described with respect to immunoassays using the antigen-antibody immunological pair in which the immobilized receptor is a protein antigen prepared by protein blotting. The liquid sample is biologically derived, (e.g., urine or serum) and the one reagent comprises a labelled antibody.

The labelled antibody or antigen described with respect to such an assay may be any of the conventional types including radioactive, enzyme, or a metal complex label which are conjugated to the antibody. Formation of conjugates between such immunological substances and labels are well known, e.g., (a) radioactive labels - U.S. 3,646,346, Hunter et al., Nature 142 (1962), 945, (b) enzyme labels-U.S. 3,654,090, 3,791,931 and 3,817,838, Wilson et al., Immunoflourescense and Related Staining Techniques, Knapp., W. et al., Eds. L. Sevier-North Holland, Bio-Medical Press, New York-Amsterdam, 1978, pp. 215-224; (c) fluorescent quencher labels - U.S. 3,996,345; (d) radioactive labels - U.S. 4,062,733; (e) fluorescent or enzyme labels U.S. 4,067,959; (f) chemiluminescent labels - U.S. 4,104,029; (g) non-enzymatic catalyst label - U.S. 4,160,645; (h) enzyme pair labels - U.S. 4,233,402, chemically induced fluorescent labels - 4,720,450; and (i) enzyme non-ionic charge labels - U.S. 4,287,300. In addition, the labels disclosed in U.S. 4,366,241 may be employed. Also, colloidal gold labels are discussed in detail hereinafter.

Colloidal gold conjugates useful for probes such as cytochemical markers are well known for microscopy. See, e.g., Scanning Electron Microscopy, 1981, II, pp. 9-31, "Immunocytochemistry" Eds. Polak, J.N., et al., Bristol, London, Boston (1982) pp. 82-112, and Journal of Neuroscience Methods, 7(1983), pp.1-18. Colloidal gold particle markers are simple to use in comparison to other conventional markers. For example, they do not require instruments necessary for detection of other markers such as radioactive isotopes. Furthermore, unlike enzymes, they do not require the additional step of adding substrate. However, they have not been used extensively for commercial immunoassay kits, perhaps because of their low level of visibility using conventional techniques for mixing reactants. For example, the sensitivity of an assay using colloidal gold conjugates in a previous membrane system has been thought to be insufficient to provide the desired level of sensitivity. It has been found that by directing the flow of the liquid sample and reagents through the membrane, the sensitivity is so improved that colloidal gold particle conjugates are useful reagents for immunoassay kits without the need to use expensive microscopes.

If the sensitivity of the gold-immunological reagent conjugate is insufficient, even with the increase of the present system, a technique for enhancing the sensitivity of the gold complex may be employed such as disclosed in Holgate, C.S., et al., J. Histochem. Cytochem 31:938 (1983) and in Dancher, G, et al., J. Histochem. Cytochem 31:1394 (1983). This system is an "indirect" technique employing an immunological reagent, immunoglobulin, absorbed to colloidal gold. The gold particles are revealed by a silver precipitation reaction. In essence, the silver enhancement takes advantage of the catalytic effect of gold to catalyze the photographic physical developer process converting silver ion to silver metal. Suitable colloidal gold or gold sol particle size is from 3nm to 150nm. This immuno gold-silver staining method may have an enhanced sensitivity of up to 200-fold in comparison to the use of the gold particles without silver staining.

The present invention is applicable to the competitive binding technique. In such system for the detection of antibody in a liquid sample, the corresponding member of the immunological pair, i.e., antigen is blotted on to the membrane surface. Antibody labelled in the manner described above of the same immunological character as the antibody analyte to be detected in the sample is contacted with the immobilized antigen on the membrane. The immobilized antigen is in limited supply, and so a competition is set up between the antibody in the sample and the labelled antibody. Thus, the signal emitted from the label is inversely proportional to the amount of antibody in the sample. The competitive binding assay may be performed by reversing the roles of the antigen and antibody. It should be understood that the system is also useful for other immunoassays such as, for example, described in U.S. Patent No. 4,366,241.

The substances to be analyzed include a wide variety of biologically derived substances, e.g., proteins. The following is a list of some of these substances. (The listed substances also include immunologically reactive antibodies, and fractions of the substances).

### Immunoglobulins

IgE
IgA
IgM
IgD

### Microorganisms

Aerobacter aerogenes
Aerobic Spore-Forming Bacilli
Bacillus anthracis
Bacillus subtilis
Bacillus cereus
Anaerobic Spore-forming Bacilli
Clostridium botulinum
Clostridium tetani
Clostridium perfringens
Brucellae
Brucella melitensis
Brucella abortus
Brucella suis
Chlamydia (unclassifiable parasites bacterial/viral)
Chlamydia agents (naming uncertain))
Chlamydia trachomatis
Corynebacteria
Corynebacterium diptheriae
Escherichia coli
Fungi
Cryptococcus neoformans
Histoplasma capsulatum
Coccidioides immitis
Candida albicans
Mucor corymbifer (absidia corymbifera)
Hemophilus-Pordetella group
Hemophilus influenzae
H. ducreyi
H. hemophilus
H. aegypticus
H. parainfluenzae
Keibsiella pneumoniae
Mycobacteria
Mycobacterium tuberculosis hominis
Mycobacterium bovis
Mycobacterium avium
Mycobacterium leprae
Mycobacterium paratuberculosis
Mycoplasmas
Mycoplasma pneumoniae
Mycoplasma hominis
Neisseriae
Neisseria meningitidis
Neisseria gonorrheae
Other Pathogens
Listeria monocytogens
Pasteurellae
Pasteurella pestis
Pasteurella multocida
Pneumococci
Diplococcus pneumoniae
Pseudomonas aeruginosa
Rickettsiae (bacteria-like parasites)
Rickettsia prowazekii
Rickettsia mooseri
Rickettsia rickettsii
Rickettsia conori
Rickettsia australis
Rickettsia tsutsugamushi
Rickettsia burnetii
Salmonella choleraesus
Salmonella typhimurtum
Salmonella typhosa
Shigella arabinotardo
Shigella boydii
Shigella dysenteriae
Shigella flexneri
Shigella schmitzii
Shigella Sonnei
Staphylococci
Staphylococcus aureus
Staphylococcus albus
Streptococci
Streptococcus pyogenes
Groups B, C, D, F, G
The Spirochetes
Treponema pallidum
Borrelia recurrentis
Leptospira icterohemorrhagiae
Leptospira canicola
Toxoplasma gondii

### Peptide and Protein Hormones

Corticotropin (ACTH)
(adrenocorticotropic hormone)
Follicle-stimulating hormone
Luteinizing hormone
(interstitial cell-stimulating hormone)
Parathyroid hormone
Prolactin
Chorionic Gonadotropin
Insulin
Glucagon
Relaxin
Somatropin
Triiodothyronine
Thyrocalcitonin
Thyroxine

### Tissue Hormones

Angiotensin I and II
Bradykinin
Gastrin
Human placental lactogen
Secretin

### Peptide Hormones

Oxytocin
Vasopressin

### Viruses

Adinoviruses
Arboviruses
Eastern Equine Eucephalitis Virus
Western Equine Eucephalitis Virus
Sindbis Virus
Semliki Forest Virus
St. Louis Encephalitis Virus
California Encephalitis Virus
Colorado Tick Fever Virus
Yellow Fever Virus
Dengue Virus
Hepatitis
Hepatitis A Virus
Hepatitis B Virus
Herpes Viruses
Herpes simplex, Types I and II
Varicella (Chicken pox)
Cytomegalovirus
Myxoviruses
Influenza (A, B, and C
Parainfluenza (1-4)
Mumps virus
Newcastle Disease Virus
Measles Virus
Canine Distemper Virus
Respiratory Syncytial Virus
Rubella Virus
Picornaviruses
Poliovirus
Coxsackievirus
Echoviruses
Rhinoviruses
Pox Viruses
Vaccinia
Molluscum contagiosum
As mentioned above, in the protein blot the immunological reagent is concentrated on at least one defined region on the membrane which appears as a dot. The reaction with the sample component and with the labelled reagent only occurs in that region.

This approach has certain advantages such as the performance of multiple simultaneous assays with a single device as described below. Also it provides a more distinctive end product signal since it is concentrated at a single region.

Another advantage of the dot approach is that it permits the simultaneous detection of multiple components in a sample. For example, with a single assay device, the simultaneous detection of more than two components of the liquid sample is permitted by a corresponding number of immobilized immunological reagent on the membrane.

There are a number of advantages to using the device of the present invention. One advantage is the use of an elongate strip membrane and elongate port in registry with it. This adapts the apparatus to analysis of elongate separated proteins such as Western blots. Also, the use of spaced multiple dot blots permits linear variations in the concentrations of sample applied to the membrane merely be tilting the device and applying the sample to the upper end of the elongate port. The dots at the lower end receive higher concentrations than those at the top.

The standard protocols for the conventional immunoassays may be used in the present invention. For example, the order of addition of the sample and labelled reagents may be simultaneous or sequential.

While monoclonal antibodies have known advantages and purity over polyclonal antibodies, either type of immunological reagent can be used in accordance with the present invention.

While the above system is described in terms of yes-no quantitative test, it should be understood that it is also suitable as semi-quantitative test using appropriate signals, such as colors, produced at different known concentrations of the component to be analyzed.

The present invention makes use of a flow-through device for the detection of antibody in an unknown sample, specifically serum, to a Western blot protein which had been previously immobilized on a membrane strip. For example, such Western blots of HIV lysate on recombinant protein are available commercially under the trademark (1) Biotech/Dupont HIV Western Blot Kit from E.I. Dupont DeNemours & Co., Inc., Wilmington, Delaware, (2) NOVOPATH^{tm} strips from BioRad, Hercules, California, or (3) EPIblot-HIV Western Blot strips from Epitope, Inc., Beaverton, Oregon. The ability to use a flow-through device rather than an overlay leads to extraordinary savings in technician time and laboratory equipment. More specifically, a flow-through AIDS test can be performed in substantially less than one hour. More specifically less than ten minutes to as short a time as five minutes without the necessity of mechanical agitation used in an overlay procedure.

Another aspect of the invention is the discovery that the use of paper backed nitrocellulose has certain significant advantages. For example, in a test for antibody using an HIV Western blot, using colloidal gold conjugate, there is a substantial increase in the differential between the intensity of the positive and the background intensity (termed "net intensity").

Another aspect of the invention is the use of certain blocking reagents for such HIV Western blot significantly increases the net intensity. Specifically, the use of Tween-20® surfactant at a concentration of 1% to 2% in solution dramatically increases such net intensity. An optimal amount of such Tween-20® is on the order of 1.5%. The performance is further increased by the addition of a conventional non-interfering blocking protein such as bovine serum albumin (BSA), gelatin or casein to the blocking solution. Preferably the bovine serum albumin should be present in a concentration of about 0.5% to about 1.5% of the blocking solution.

A generalized procedure suitable for performing the method of the present invention to detect antibodies in a patient's serum against an HIV Western blot is as follows. First, a strip containing the HIV Western blot is placed into the device of Figures 1-4 on top of the absorbent material. Then the top wall is slid over the bottom wall so that rim 16 is pressed downwardly against the top surface of paper-backed nitrocellulose membrane 18. With the side walls of the bottom wall nested with the corresponding side walls of the top wall, U-shaped members 24 are used to retain the system in a liquid-tight, box-like enclosure.

Then, a blocking solution is applied to membrane 16. In this instance, the solution is applied to the upper surface of the membrane and is drawn through it under the influence of absorbent body 20. As set forth above, a particularly effective blocking solution is water-based and includes Tween-20® and 1% to 2% of a non-interfering blocking protein such as BSA, casein or gelatin, at a concentration of about 0.5% to about 1.5%.

Then, the sample in the form of undiluted patient serum is applied to the top of the membrane. The sample flows through the membrane in about 15 to 30 seconds.

Then, the membrane is washed by passing washing solution in about 15 to 30 seconds. The washing solution may comprise the same solution as the blocking solution or any other known washing solution such as a low ionic strength buffer (e.g. less than 20 mM) or deionized water.

Then, a labelled conjugate reactive with the selected antibody in the patient's serum is added. In a preferred embodiment, the conjugate is of protein A with colloidal gold as a label. Alternatively, other labels may be employed for certain applications including enzyme or a radio active tag.

In a final step, another washing solution is applied to the membrane.

A specific example of the practice of the present invention are set forth in the following example by way of illustration.

### Example 1

This is a Western blot assay using the assay device of Figures 1-4.

HIV-1 viral lysate containing 800 micrograms/ml of viral proteins were mixed with an equal volume of 0.1 M Tris-HCl buffer at pH 6.8 containing 2% SDS, 20% sucrose, and 0.01% bromophenol blue tracking dye. 40 microliter of this solution was loaded into a well of a Laemmeli SDS-polyacrylamide gel (10% acrylamide). The electrophoretic buffer system and running conditions were those of Laemmeli (Laemmeli, 1970 Nature vol. 227, p.680-685).

When the tracking dye reached the bottom of the gel, the electrophoretic run was terminated. The gel was removed and washed as described in the BIO-RAD Transblot manual. (BIO-RAD Laboratories, Inc., Hercules, California) The protein in the gel was then transferred onto paper-backed nitrocellulose using the BIO-RAD transblot apparatus. The protocol and reagents used were those described in the Transblot operating manual. Electrophoretic blotting was carried out overnight at a constant voltage of 30V.

The paper-backed nitrocellulose to which the blot was transferred was then placed between two absorbent filter papers and allowed to dry overnight. A strip from the sheet corresponding to the lane in which there were viral proteins was cut and mounted into the filtration device of Figures 1-4.

0.5 ml of a buffer containing 2% v/v Tween-20® in 10 mM phosphate at pH 7.4 and 0.15M sodium chloride and 1% w/v bovine serum albumin was added and allowed to flow through the membrane to block off the remaining reactive sites. 0.3 ml of a seropositive human serum sample (control material from Biotech Research Laboratories, Rockville, MD) was added and allowed to flow through the membrane. 0.3 ml of the same buffer used to wash the membrane was then added and allowed to flow through followed by 0.3 ml of deionized water. 0.4 ml of a solution of Protein-A colloidal gold was then added and allowed to flow through the membrane. (Protein-A colloidal gold with a particle size of 15 nm and at an optical density of 2.5 at wavelength 520 nm is available from E-Y Laboratories, Inc.). 0.4 ml of deionized water was then allowed to flow through the membrane as a final wash. The major bands required to indicate seropositivity was revealed as distinct red bands. The entire procedure, from time of adding the first buffer solution to the development of the bands, took five minutes.

## Claims

1. A storage and reaction apparatus for use in assays for the detection and/or determination of a bindable target substance in a liquid sample suspected of containing such substance, characterised in that it comprises
(a) a liquid-permeable, porous reaction membrane strip having an upper and lower surface, at least said upper surface having immobilized thereon a receptor capable of directly or indirectly binding to the bindable substance, said receptor comprising a protein blot along said strip,
(b) a body of absorbent material capable of absorbing liquid, said body having a surface located adjacent to the lower surface of the reaction membrane, and
(c) container means for said reaction membrane and absorbent material, including a top wall defining a fluid port in the form of an elongate slot adjacent said receptor and including fluid seal means disposed at the periphery of the fluid port defining a seal between said container top wall and reaction membrane, said top wall and membrane defining an open space to the periphery of said seal means.

2. The storage and reaction apparatus of claim 1 characterised in that said seal means comprises a continuous rim projecting from said container top wall towards said reaction membrane, said rim and reaction membrane being in contact under sufficient compression to prevent any substantial leakage between said rim and reaction membrane.

3. The storage and reaction apparatus of claim 2 characterised in that said rim contacts said strip at a location to leave a portion of the sides of the strip exposed to said open space.

4. The storage and reaction apparatus of claim 2 or claim 3 wherein said rim has an outer wall projecting to an area of said strip interior of the periphery of the strip thereby forming an open area on the top of the strip outside the rim and free of contact with the rim.

5. The storage and reaction apparatus of claim 3 or 4 characterised in that said target substance comprises antibody.

6. The storage and reaction apparatus of any one of claims 2 to 5 characterised in that said rim is less than about 5mm in width.

7. The storage and reaction apparatus as claimed in any preceding claim characterised in that said porous membrane comprises nitrocellulose.

8. The storage and reaction apparatus as claimed in any preceding claim characterised in that said porous membrane comprises a nitrocellulose membrane bonded to at least one liquid-permeable support material facing said absorbent material body.

9. The storage and reaction apparatus of claim 8 characterised in that said support material is paper, fiberglass or polyester.

10. The storage and reaction apparatus of any preceding claim characterised in that said reaction membrane rests on said absorbent material body and is readily removable from said container means.

11. The storage and reaction apparatus of any preceding claim characterised in that said container top wall is detachably mounted to said container means.

12. An assay for the detection and/or determination of bindable target substance in a liquid sample suspected of containing such substance characterised in that it employs as an assay device storage and reaction apparatus as described in any one of claims 1 to 11, said assay comprising applying the sample, a visable labelled substance and any additional reagents to the upper surface of said permeable reaction membrane whereby the applied liquid will permeate through said fluid port and the membrane lower surface into said absorbent body, and thereafter detecting said labelled substance bound to the upper surface as an indication of the presence or absence of bindable target substance bound to said receptor of said upper surface.

13. The assay of claim 12 characterised in that said label comprises colloidal gold.

14. The assay of claim 12 or 13 characterised in that said protein blot comprises HIV protein.

15. The assay of claim 14 in which said HIV protein comprises recombinant HIV protein or virus lysate.

16. The assay of any one of claims 12 to 15 characterised in that a blocking agent is added to the reaction membrane prior to addition of said labelled substance comprising between 1% and 2% Tween 20® surfactant.

17. The assay of any one of claims 12 to 15 characterised in that a blocking agent is added to the reaction membrane prior to the addition of said labelled substance which comprises a combination of surfactant and a non-interfering blocking protein serum albumin.

18. The assay of claim 16 characterised in that said blocking agent further comprises between about 0.5% and about 1.5% non-interfering protein.

19. The assay of claim 18 characterised in that said non-interfering protein comprises bovine serum albumin.

## Patentansprüche

1. Vorrichtung zur Aufbewahrung und Umsetzung zur Verwendung bei Analysen zum Nachweis und/oder zur Bestimmung einer bindungsfähigen Targetsubstanz in einer Flüssigkeitsprobe, von der angenommen wird, daß sie diese Substanz enthält, dadurch **gekennzeichnet,** daß sie
a) einen flüssigkeitsdurchlässigen porösen Streifen als Reaktionsmembran mit einer oberen und unteren Oberfläche, von denen wenigstens die obere Oberfläche einen darauf immobilisierten Rezeptor aufweist, der zur direkten oder indirekten Bindung an die bindungsfähige Substanz befähigt ist und einen Proteinfleck auf dem Streifen umfaßt,
b) einen Körper aus einem absorbierenden Stoff, der zur Absorption von Flüssigkeit befähigt ist, wobei der Körper eine an die untere Oberfläche der Reaktionsmembran anschließende Oberfläche aufweist, und
c) ein Gefäß für die Reaktionsmembran und den absorbierenden Stoff, das eine obere Wand, welche einen Flüssigkeitseinlaß in Form eines verlängerten, an den Rezeptor anschließenden Schlitzes begrenzt und Flüssigkeitsabdichtmittel umfaßt, die am Umfang des Flüssigkeitseinlasses angeordnet sind, der eine Dichtung zwischen der oberen Gefäßwand und der Reaktionsmembran begrenzt, wobei die obere Wand und die Membran einen zum Umfang des Abdichtmittels hin offenen Raum begrenzen, umfaßt.

2. Vorrichtung zur Aufbewahrung und Umsetzung nach Anspruch 1, dadurch **gekennzeichnet,** daß das Dichtmittel eine geschlossene Umrandung umfaßt, die von der oberen Wand des Gefäßes bis zur Reaktionsmembran vorspringt, wobei die Umrandung und die Reaktionsmembran unter ausreichendem Druck zur Verhinderung einer starken Undichtheit zwischen der Umrandung und der Reaktionsmembran miteinander in Kontakt stehen.

3. Vorrichtung zur Aufbewahrung und Umsetzung nach Anspruch 2, dadurch **gekennzeichnet,** daß die Umrandung den Streifen an einer Stelle so berührt, daß ein Teil der Seitenflächen des Streifens dem offenen Raum gegenüber ausgesetzt ist.

4. Vorrichtung zur Aufbewahrung und Umsetzung nach Anspruch 2 oder 3, wobei die Umrandung eine Außenwand aufweist, die bis zu einem Bereich des Streifens innerhalb seiner Umgebung vorspringt und dadurch einen offenen Bereich auf der Oberseite des Streifens bildet, der außerhalb der Umrandung liegt und diese nicht berührt.

5. Vorrichtung zur Aufbewahrung und Umsetzung nach Anspruch 3 oder 4, dadurch **gekennzeichnet,** daß die Targetsubstanz einen Antikörper umfaßt.

6. Vorrichtung zur Aufbewahrung und Umsetzung nach einem der Ansprüche 2 bis 5, dadurch **gekennzeichnet,** daß die Umrandung eine Breite von weniger als ca. 5 mm aufweist.

7. Vorrichtung zur Aufbewahrung und Umsetzung nach einem der vorangegangenen Ansprüche, dadurch **gekennzeichnet,** daß die poröse Membran Nitrocellulose umfaßt.

8. Vorrichtung zur Aufbewahrung und Umsetzung nach einem der vorangegangenen Ansprüche, dadurch **gekennzeichnet,** daß die poröse Membran eine Nitrocellulosemembran umfaßt, die mit wenigstens einem flüssigkeitsdurchlässigen, den Körper aus absorbierendem Stoff bekleidenden Substrat verbunden ist.

9. Vorrichtung zur Aufbewahrung und Umsetzung nach Anspruch 8, dadurch **gekennzeichnet,** daß das Substrat Papier, Glasfasern oder Polyester ist.

10. Vorrichtung zur Aufbewahrung und Umsetzung nach einem der vorangegangenen Ansprüche, dadurch **gekennzeichnet,** daß die Reaktionsmembran auf dem Körper aus absorbierendem Stoff aufsitzt und vom Gefäß rasch entfernt werden kann.

11. Vorrichtung zur Aufbewahrung und Umsetzung nach einem der vorangegangenen Ansprüche, dadurch **gekennzeichnet,** daß die obere Wand des Gefäßes auf diesem abnehmbar angebracht ist.

12. Analyse zum Nachweis und/oder zur Bestimmung einer bindungsfähigen Targetsubstanz einer Flüssigkeitsprobe, von der angenommen wird, daß sie diese Substanz enthält, dadurch **gekennzeichnet,** daß dabei eine Analysenvorrichtung zur Aufbewahrung und Umsetzung nach einem der Ansprüche 1 bis 11 verwendet wird, wobei die Analyse die Aufbringung der Probe, einer sichtbar markierten Substanz und beliebiger zusätzlicher Reagenzien auf die obere Fläche der durchlässigen Reaktionsmembran, wodurch die aufgebrachte Flüssigkeit durch den Flüssigkeitseinlaß und die untere Fläche der Membran in den Körper des Absorbens eindringt, und den anschließenden Nachweis der mit der oberen Fläche verbundenen markierten Substanz als Indiz für die Anwesenheit oder Abwesenheit der mit dem Rezeptor der oberen Fläche verbundenen bindungsfähigen Targetsubstanz umfaßt.

13. Analyse nach Anspruch 12, dadurch **gekennzeichnet,** daß die Markierung kolloidales Gold darstellt.

14. Analyse nach Anspruch 12 oder 13, dadurch **gekennzeichnet,** daß der Proteinfleck HIV-Protein darstellt.

15. Analyse nach Anspruch 14, bei der das HIV-Protein rekombinantes HIV-Protein oder Viruslysat umfaßt.

16. Analyse nach einem der Ansprüche 12 bis 15, dadurch **gekennzeichnet,** daß der Reaktionsmembran vor der Zugabe der markierten Substanz ein Blockierungsmittel zugesetzt wird, das zwischen 1 und 2% Tween 20® als Tensid umfaßt.

17. Analyse nach einem der Ansprüche 12 bis 15, dadurch **gekennzeichnet,** daß der Reaktionsmembran vor der Zugabe der markierten Substanz ein Blockierungsmittel zugesetzt wird, das ein Gemisch aus einem Tensid und einem keinen störenden Einfluß ausübenden blockierenden Proteinserumalbumin darstellt.

18. Analyse nach Anspruch 16, dadurch **gekennzeichnet,** daß das Blockierungsmittel außerdem noch zwischen ca. 0,5 und ca. 1,5% eines keinen störenden Einfluß ausübenden Proteins umfaßt.

19. Analyse nach Anspruch 18, dadurch **gekennzeichnet,** daß das keinen störenden Einfluß ausübende Protein Rinderserumalbumin darstellt.

## Revendications

1. Appareil d'accumulation et de réaction destiné à être utilisé dans des analyses pour la détection et/ou la détermination d'une substance cible à capacité de liaison dans un échantillon liquide suspecté de contenir une telle substance, caractérisé en ce qu'il comprend
(a) une bandelette de membrane réactionnelle poreuse perméable aux liquides, possédant une surface supérieure et une surface inférieure, au moins ladite surface supérieure portant un récepteur immobilisé capable de se lier directement ou indirectement à la substance à capacité de liaison, ledit récepteur comprenant une tache de protéine le long de ladite bandelette,
(b) une masse de matière absorbante capable d'absorber un liquide, ladite masse possédant une surface située en position adjacente à la surface inférieure de la membrane réactionnelle, et
(c) un récipient pour ladite membrane réactionnelle et ladite matière absorbante, comprenant une paroi supérieure définissant un orifice d'écoulement de fluide sous forme d'une fente allongée adjacente audit récepteur et comprenant un moyen étanche aux fluides disposé à la périphérie de l'orifice d'écoulement de fluide, définissant un joint entre ladite paroi supérieure du récipient et ladite membrane réactionnelle, ladite paroi supérieure et ladite membrane définissant un espace ouvert à la périphérie dudit moyen étanche.

2. Appareil d'accumulation et de réaction suivant la revendication 1, caractérisé en ce que le moyen étanche comprend une nervure continue faisant saillie de la paroi supérieure du récipient vers la membrane réactionnelle, ladite nervure et ladite membrane réactionnelle étant en contact par une compression suffisante pour empêcher une quelconque fuite notable entre ladite nervure et ladite membrane réactionnelle.

3. Appareil d'accumulation et de réaction suivant la revendication 2, caractérisé en ce que la nervure entre en contact avec la bandelette à un endroit laissant une portion des côtés de la bandelette en contact avec l'espace ouvert.

4. Appareil d'accumulation et de réaction suivant la revendication 2 ou la revendication 3, dans lequel la nervure possède une paroi extérieure faisant saillie à une zone de l'intérieur de la bandelette, à la périphérie de la bandelette, en formant ainsi une zone ouverte sur le dessus de la bandelette à l'extérieur de la nervure et sans contact avec la nervure.

5. Appareil d'accumulation et de réaction suivant la revendication 3 ou 4, caractérisé en ce que la substance cible comprend un anticorps.

6. Appareil d'accumulation et de réaction suivant l'une quelconque des revendications 2 à 5, caractérisé en ce que la nervure possède une largeur inférieure à environ 5 mm.

7. Appareil d'accumulation et de réaction suivant l'une quelconque des revendications précédentes, caractérisé en ce que la membrane poreuse comprend de la nitrocellulose.

8. Appareil d'accumulation et de réaction suivant l'une quelconque des revendications précédentes, caractérisé en ce que la membrane poreuse consiste en une membrane de nitrocellulose liée à au moins une matière de support perméable aux liquides faisant face à la masse de matière absorbante.

9. Appareil d'accumulation et de réaction suivant la revendication 8, caractérisé en ce que la matière de support consiste en papier, fibre de verre ou polyester.

10. Appareil d'accumulation et de réaction suivant l'une quelconque des revendications précédentes, caractérisé en ce que la membrane réactionnelle repose sur la masse de matière absorbante et peut être enlevée aisément du récipient.

11. Appareil d'accumulation et de réaction suivant l'une quelconque des revendications précédentes, caractérisé en ce que la paroi supérieure du récipient est montée de manière amovible sur ledit récipient.

12. Analyse pour la détection et/ou la détermination d'une substance cible à capacité de liaison dans un échantillon liquide suspecté de contenir cette substance, caractérisée en ce qu'elle utilise comme dispositif analytique l'appareil d'accumulation et de réaction décrit dans l'une quelconque des revendications 1 à 11, ladite analyse comprenant l'application de l'échantillon, d'une substance marquée visualisable et de n'importe quels réactifs supplémentaires à la surface supérieure de la membrane réactionnelle perméable, ce qui permet aux liquides appliqués de passer à travers l'orifice d'écoulement de fluide et la surface inférieure de la membrane dans la masse d'absorbant, puis la détection de ladite substance marquée liée à la surface supérieure comme indication de la présence ou de l'absence de la substance cible à capacité de liaison liée au récepteur de ladite surface supérieure.

13. Analyse suivant la revendication 12, caractérisée en ce que le marqueur comprend de l'or colloïdal.

14. Analyse suivant la revendication 12 ou 13, caractérisée en ce que la tache de protéine comprend une protéine de HIV.

15. Analyse suivant la revendication 14, dans laquelle la protéine de HIV consiste en une protéine de HIV recombinante ou un lysat de virus.

16. Analyse suivant l'une quelconque des revendications 12 à 15, caractérisée en ce qu'un agent de blocage est ajouté à la membrane réactionnelle, avant addition de la substance marquée, comprenant 1 % à 2 % de surfactant Tween 20®.

17. Analyse suivant l'une quelconque des revendications 12 à 15, caractérisée en ce qu'un agent de blocage est ajouté à la membrane réactionnelle, avant addition de la substance marquée, qui comprend une association d'un surfactant et d'une protéine de blocage non interférente consistant en sérum-albumine.

18. Analyse suivant la revendication 16, caractérisée en ce que l'agent de blocage comprend en outre une quantité d'environ 0,5 % à environ 1,5% de protéine non interférente.

19. Analyse suivant la revendication 18, caractérisée en ce que la protéine non interférente consiste en sérum-albumine bovine.
